# EUROPEAN PATENT SPECIFICATION

(11) **EP 3 515 405 B1**
(45) Date of publication and mention of the grant of the patent: **18.10.2023**
(21) Application number: 17771554.7
(22) Date of filing: 15.09.2017
(51) Int. Cl.: A61K 9/70, A61K 33/40, A61K 47/08, A61K 9/08, A61L 26/00, A61P 17/00, A61P 31/10, A61P 43/00

(54) **COMPOSITION FOR THE TREATMENT OF A FUNGAL INFECTION**
ZUSAMMENSETZUNG ZUR BEHANDLUNG EINER PILZINFEKTION
COMPOSITION POUR LE TRAITEMENT D'UNE INFECTION FONGIQUE

(30) Priority: 20.09.2016 GB 201616003
(43) Date of publication of application: 31.07.2019
(73) Proprietor: Insense Limited, Sharnbrook, Bedford MK44 1LQ (GB)
(72) Inventor: DAVIS, Paul, Bedford Bedfordshire MK44 1LQ (GB); AUSTIN, Corrine, Bedford Bedfordshire MK44 1LQ (GB)
(74) Representative: Willett, Christopher David
(86) International application number: PCT/GB2017/052736
(87) International publication number: WO 2018/055341

(56) References cited:
- WO-A1-2005/072783
- WO-A2-2006/133523
- AU-A1- 2012 216 857
- US-A1- 2014 079 804

## Description

### Technical Field

The invention relates to a packaged treatment composition comprising a polymerisable and/or curable composition, particularly for treating a fungal infection of a nail of a human or animal.

### Background and Prior Art

Healthy nails in visibly good condition are important and highly prized aspects of human appearance. Frequently the appearance, strength and health of nails can be adversely affected by infection with pathogenic fungal organisms, typically of the genus Trychophyton, and there is a strong demand for therapies that improve the appearance of affected nails by elimination of the infecting fungal infection.

Onychomycosis (also known as dermatophytic onychomycosis or *tinea unguium*) is a fungal infection associated with the nail. It is the most common disease of the nails and constitutes about half of all nail abnormalities. This condition affects both toenails and fingernails, but toenail infections are particularly common and account for about 90% of all reported infections. It is estimated that the condition occurs in about 10 percent of the adult population although higher incidences have been reported in some patient populations, in particular diabetic patients (reported prevalence ~33%), psoriatic patients (reported prevalence ~18%) and immunocompromised HIV patients (reported prevalence 15-40%). The overall prevalence rate of onychomycosis is determined by several factors including age, predisposing factors, social class, occupation, climate, living environment and frequency of travel.

It is well-established that within the UK, patients do not generally present for treatment because onychomycosis is largely asymptomatic; when patients do present, it is usually for cosmetic reasons without any physical complaints, although loss of self-esteem and lack of social interaction have been reported. Increasingly however, onychomycosis is being viewed as more than a mere cosmetic problem. Despite improved personal hygiene and living environments, onychomycosis continues to spread and persist; moreover, it is an infection that does not resolve spontaneously. Indeed, infection can worsen, spread to other uninfected locations (e.g. other nails or to the surrounding skin) and infect other individuals. Infections of the toenail therefore have the potential to affect the quality of life of sufferers. This is of particular importance in high risk patients such as those with a compromised immune system or in diabetics, for example where it can increase the risk for other foot disorders and limb amputation. Morbidity resulting from onychomycosis, particularly in severe cases, includes interference with standing, walking, and exercising. In addition, infected persons may report paresthesia, pain, discomfort, and loss of dexterity.

Onychomycosis is caused by 3 main classes of fungi: dermatophytes, yeasts, and non-dermatophyte moulds. Dermatophytes are considered to be by far the most common cause of onychomycosis. Two major pathogens are considered responsible for approximately 90% of all onychomycosis cases in Europe. *Trichophyton rubrum* accounts for 70% and *Trichophyton mentagrophytes* accounts for 20% of all cases. Onychomycosis caused by non-dermatophyte molds (*Fusarium* species, *Scopulariopsis brevicaulis, Aspergillus* species) is becoming more common worldwide, accounting for up to 10% of cases. Onychomycosis due to *Candida* is generally considered rarer. Studies in Brazil identified yeasts in 52% of positive cultures (*Candida albicans* 18.3%, *Candida parapsilosis* 13.8%, other species of Candida 15.4% and other yeasts 4.6%), followed by dermatophytes in 40.6% of positive cultures (the most commonly isolated organisms were *Trichophyton rubrum* in 33.2%, followed by Trichophyton *mentagrophytes* in 6.3% and others 1.2%). Non-dermatophyte moulds were isolated in 7.4% of positive cultures (*Fusarium spp.* 4.5%, *Nattrassia mangiferae* 2.3% and *Aspergillus spp.* 0.6%).

Although there are numerous remedies on the market, there is widespread dissatisfaction with available technologies and products because the active ingredients do not readily penetrate the nail and little, if any, of the material applied to the top surface reaches the underlying structures where fungal cells can reside in relative safety.

Systemically delivered agents can reach the nail region through the blood stream, but poor penetration into the nail region from the circulation and serious side effects limit the usefulness of the approach.

Fungally infected nails are often rendered porous or open by the action of the invading fungi. Thus, often the nail is co-colonised with bacteria which can exacerbate the damaging effects of the fungi by releasing additional destructive enzymes and locally active toxins.

It is well recognised that even if a fungal nail infection is reduced by a known therapy, it is seldom completely eliminated and it is usual for infections to return soon after treatment is stopped.

WO2010/125358 discloses a two-part treatment method, wherein an aqueous liquid is first applied to the nail, followed by a dressing capable of delivering hydrogen peroxide. US2009/0232876 discloses a composition for forming an in-situ blockage of a wound to control bleeding and comprises hydrogen peroxide, a polymer-forming component and a decomposing agent for the hydrogen peroxide.

AU2012216857 discloses a hydrogen peroxide delivery system for delivering hydrogen peroxide to a part of a human or animal body, comprising, prior to use, a component comprising hydrogen peroxide in dry condition, and a component in hydrated condition, such that, in use, when the components are placed in contact with each other and arranged with the hydrated component nearer the skin than the dry hydrogen peroxide component, hydrogen peroxide migrates towards the hydrated component.

Thus, improvements in this area would be highly desirable.

### Summary of the Invention

The invention relates to a packaged treatment composition comprising a polymerisable and/or curable composition comprising (1) a packaged first flowable component comprising a source of hydrogen peroxide and (2) a separately packaged second flowable component; the composition comprising a polymerisable monomer or oligomer, a polymer and/or a curing agent, wherein the composition comprises a photoinitiator and wherein the first flowable component is substantially free of photoinitator, the composition capable of forming a solid composition by UV-initiated polymerisation and/or curing following mixing of the first and second flowable components.

Such a composition can be dispensed onto the surface of a nail in need of treatment for fungal infection. The composition comes as two separate components (to be mixed together either immediately before application to the nail or while in place on the nail surface).

Subsequent to delivery on the nail surface, and following mixing of the two components, polymerisation, curing, or both, of the composition occurs, in order to provide a solid composition which will remain in place on the nail as treatment takes place.

In one embodiment the composition comprises a polymerisable composition comprising a polymerisable monomer or oligomer. The monomer or oligomer can then be polymerised to form the solid composition.

As hydrogen peroxide may be reactive with a variety of monomers or oligomers, it may be preferable that the first flowable component is substantially free of polymerisable monomer or oligomer.

However, it may be preferable that the second flowable component is substantially free of polymerisable monomer or oligomer for formulation reasons.

In another embodiment the composition comprises a curable composition comprising a polymer and a curing agent. In this embodiment the composition already comprises a polymer and the solid composition is formed upon curing of the polymer. Typically the curing agent is a cross-linking agent in order to provide cross-links between the polymer chains, as this helps to provide a solid structure.

As hydrogen peroxide may be reactive with a variety of curing agents it may be preferable that the first flowable composition is substantially free of curing agent.

Of course the composition can comprise a polymerisable composition, a curable composition or a composition that is both polymerisable and curable.

The polymerisation reaction and/or curing, occurs by the application of ultra violet light (UV) and is triggered by the photoinitiator. The two flowable components can be mixed and placed on the nail surface without any curing or polymerisation occurring. Only once in place and well-mixed is polymerisation initiated when a UV source is introduced to the mixed composition. As hydrogen peroxide may be reactive with a variety of photoinitiators it may be preferable that the first flowable composition is substantially free of photoinitiator.

Thus, preferably the packaged composition is accompanied by a UV source.

The source of hydrogen peroxide is preferably pre-formed hydrogen peroxide. The source of hydrogen peroxide may comprise hydrogen peroxide *per se* or hydrogen peroxide in combination with or complexed with another entity. Alternatively the source of hydrogen peroxide may be a hydrogen peroxide generation means.

Preferably the polymerisation and/or curing reaction produces a hydrated hydrogel which is formed from polymerised chains cross-linked together to form the hydrogel.

Suitable hydrated hydrogels are disclosed in WO 03/090800. The hydrated hydrogel conveniently comprises hydrophilic polymer material. Suitable hydrophilic polymer materials include polyacrylates and methacrylates, e.g. as supplied by First Water Ltd in the form of sheet hydrogels, including poly 2-acrylamido-2-methylpropane sulphonic acid (polyAMPS) or salts thereof (e.g. as described in WO 01/96422), polysaccharides e.g. polysaccharide gums particularly xanthan gum (e.g. available under the Trade Mark Keltrol), various sugars, polycarboxylic acids (e.g. available under the Trade Mark Gantrez AN-169 BF from ISP Europe), poly(methyl vinyl ether co-maleic anhydride) (e.g. available under the Trade Mark Gantrez AN 139, having a molecular weight in the range 20,000 to 40,000), polyvinyl pyrrolidone (e.g. in the form of commercially available grades known as PVP K-30 and PVP K-90), polyethylene oxide (e.g. available under the Trade Mark Polyox WSR-301), polyvinyl alcohol (e.g. available under the Trade Mark Elvanol), cross-linked polyacrylic polymer (e.g. available under the Trade Mark Carbopol EZ-1), celluloses and modified celluloses including hydroxypropyl cellulose (e.g. available under the Trade Mark Klucel EEF), sodium carboxymethyl cellulose (e.g. available under the Trade Mark Cellulose Gum 7LF) and hydroxyethyl cellulose (e.g. available under the Trade Mark Natrosol 250 LR).

Mixtures of hydrophilic polymer materials may be used in a gel.

In a hydrated hydrogel of hydrophilic polymer material, the hydrophilic polymer material is desirably present at a concentration of at least 0.1%, preferably at least 0.5%, preferably at least 1%, preferably at least 2%, more preferably at least 5%, yet more preferably at least 10%, or at least 20%, desirably at least 25% and even more desirably at least 30% by weight based on the total weight of the gel. Even higher amounts, up to about 40% by weight based on the total weight of the hydrogel, may be used.

A preferred hydrated hydrogel comprises poly 2-acrylamido-2-methylpropane sulphonic acid (poly AMPS) or salts thereof, preferably in an amount of about 20% by weight of the total weight of the gel.

Typically the two compositions are both aqueous solutions. In a preferred arrangement one, or preferably both, components are in gel form.

Preferably the components have a predetermined viscosity so that in use they have a "thickened" consistency, allowing them to flow from the packaging but to stand up on the nail surface. Preferably both the first and second flowable components have substantially the same viscosity as this aids mixing of the two. By "substantially the same viscosity" is meant that the ratio of the viscosities of the two components at a shear rate of 0.1s⁻¹ at a temperature of 20°C is less than 2: 1.

One convenient way to achieve the desired similar viscosity is to include thickeners in one or both of the flowable components. Preferably the thickener is a polymeric thickening agent.

In a convenient arrangement the packaging is arranged to dispense a predefined amount of each of the first and second components. The predefined amounts will generally be determined according to the relative quantities of the components involved in the polymerisation reaction, in order that it proceeds at the desired rate and extent. However, preferably the predetermined amounts are wherein approximately equal volumes of the two components are dispensed. By "approximately equal volume" is meant that the ratio of the volumes of the two components dispensed is less than 2: 1.

When the packaging is arranged to dispense a predefine amount of each of the first and second components and the hydrogen peroxide is present as preformed hydrogen peroxide, the concentration of pre-formed hydrogen peroxide in the combined mixture can be controlled. Thus preferably the hydrogen peroxide is present at a concentration of from 0.2 to 1.5 wt% of the total of the predefined amount of the first and second compositions. Such levels overcome the two basic problems that have previously blocked successful use of hydrogen peroxide for the purpose of treating fungal nail infections i.e. achieving a sustained effective dose over several hours and controlling the dose-rate such that it is high enough to kill the fungi but not high enough to form oxygen bubbles between the nail bed and nail plate.

Hydrogen peroxide decomposes exothermally in the presence of certain catalytically acting impurities, to form oxygen gas and water. The stability of hydrogen peroxide solutions is therefore influenced primarily by the temperature, the pH value, and above all by the presence of impurities with a decomposing effect. An increase in the temperature promotes the decomposition as well as a higher pH value. For optimum stability, the pH range of pure hydrogen peroxide is typically below 4.5. Above pH 5, the decomposition increases sharply.

Therefore, commercial solutions are generally adjusted to a pH value below 5. To further aid stability, stabilizers, are added to commercial grades in ppm amounts.

In a preferred embodiment the packaging provides for mixing of the predefined amount of the first and second compositions prior to dispensing. This may, for example, by providing for a mixing chamber in the packaging or allowing the two chambers to come into fluid communication, allowing mixing prior to dispensing.

There are several ways to achieve practical delivery of the respective monomer or oligomer formulations. They can be packaged in individual syringes, or single-use dual-syringes. Such syringes could enable homogenous mixing of the two component gels prior to dispensing and at the same time, facilitate application to the nail by an individual user.

Another preferred form of packaging involves the first and second components being stored in respective first and second flexible compartments joined together by a breakable seal. A preferred form of packaging utilises flexible pouches, e.g. dual-compartment, laminated foil pouches. In this embodiment the first component is contained in a first compartment and the second component is contained in a contiguous second compartment. The two compartments are separated from each other by a seal (e.g. by a seal strip e.g. of glue, one area of which could be modified to create a zone of relative weakness) which is breakable in use to enable pressure inside the first compartment (e.g. resulting from squeezing by hand) to force the first component contained therein to mix with the second component in the second compartment. Mixing is readily achieved by alternately squeezing one compartment and then the other a number of times (e.g. 10) to force the combined component gels back and forth from one compartment to other multiple times. This action is very efficient as a means of mixing within a confined and convenient container. The mixed composition can be easily applied to the nail by tearing or cutting off a corner of the pouch and gently squeezing the required amount of gel onto the middle of the intended nail or nails, ready for in-situ polymerisation by UV irradiation.

The polymerised composition, e.g. a hydrogel, thus becomes a solid patch, locked into a shape that matches the nail on which it is applied, and with an exact match between the nail's micro surface topography and the opposing face of the polymerised composition, e.g. a hydrogel, cast and cured through the application process. This provides the required adhesive grip and also enables the most efficient molecular transfer of dissolved hydrogen peroxide from the gel into the nail plate.

The relative concentrations of ingredients and extent of cross-linking serve to endow the patch with the desired physical properties and rate of hydrogen peroxide delivery. Hydrogen peroxide delivered in this way is able to kill infecting fungi and bacteria without causing painful side effects to the user.

This novel technology provides controlled, sustained delivery of topical hydrogen peroxide to infected nails. In essence, the invention transforms the pharmacokinetic profile of hydrogen peroxide so that the new profile is perfectly tuned for delivery of an optimised dose of active therapeutic agent in a series of 8-hour (overnight) episodes of treatment while the patch is in place on the target nail. Effectively, hydrogen peroxide incorporated into the cross-linked patch becomes the perfect chemotherapeutic agent for eliminating dermatophyte infections from the nail. Free hydrogen peroxide, itself, is a broad spectrum anti-fungal agent capable of killing any of the fungi identified as organisms that infect human nails, as well as co-infecting bacteria which can also inhabit a fungally degraded nail.

The invention will be illustrated by way of example and with reference to the following figures, in which:
Figure 1 shows a suitable primary container. Single-Use Dual-syringes, which would be pre-loaded with Hydrogen Peroxide "Active" Gel and "photoinitiator gels". Mixing is obtained by fitting a mixing tip to the end of the syringe.
Figure 2 is a chart showing the release of hydrogen peroxide beneath the nail, as evidenced by colour changes in starch iodide plates.

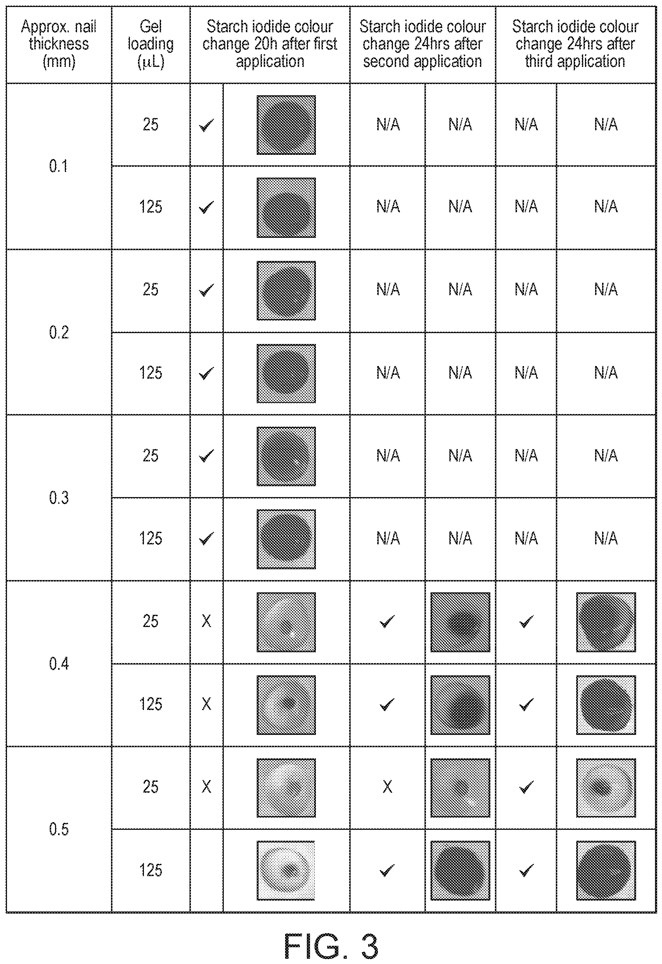

Figure 3 is a table showing the outcome of starch iodide agar sensing of through-the-nail diffusion of hydrogen peroxide after three applications of 25µL and 125µL of 0.5%w/w of a gel according to the invention.
Figure 4 is a diagrammatic representation of the experimental technique of Example 5 (not drawn to scale). In this set-up the T. rubrum is held in a much reduced volume only 0.25ml (or 0.25 gram by weight). This means that the hydrogen peroxide released by the 0.05g of gel can only be diluted by a factor of 5 while it is attacking the target T. rubrum contained in the agar. The killing efficiency can then be determined by removing the 0.25g seeded agar target and placing it onto sterile nutrient agar, so allowing any surviving T. rubrum to grow and become detectable.
Figure 5 is a table showing the fungicidal activity of hydrogen peroxide released from a dual gel (0.05g) applied to intact nail pieces. In this experiment the target T. rubrum was held in a small quantity (0.25g) of nutrient agar, separated from the gel by an intact nail piece. Hydrogen peroxide could only reach the target by passing through the nail

### Examples

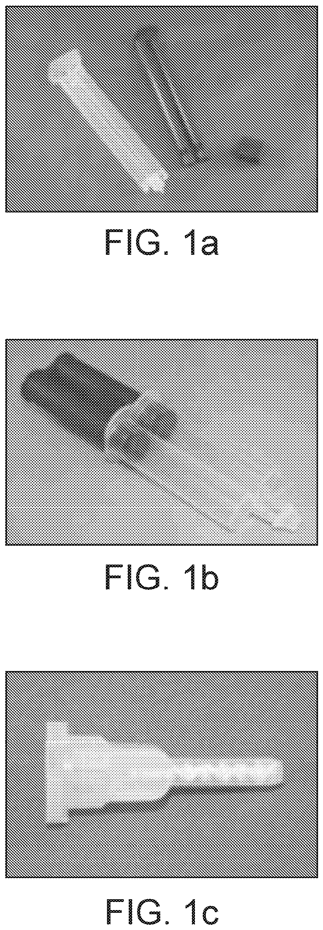

Users of the dual syringe format as shown in figures 1 a to 1c, are able to mix the contents of one "Active" Gel and one "Photo-initiator" gel by depressing the plunger on the provided dual syringe, to administer the final formulation onto the nail, based on delivering a dose of e.g. 0.15mL per cm² of mixed product. This will provide for a gel thickness of approximately 1.5mm thick. The applied gel is subsequently exposed to UV light for 1-2 mins using a standardised UV light source. This action polymerises (cures) the active polymer onto the nail and allows it to firmly adhere to the surface of the nail, thereby allowing it to remain in place overnight (i.e. for a period of 8-10 hours). Although cured to the nail, the polymerized gel (due to the presence of glycerol) remains soft and rubbery and is designed to allow the patient to peel the gel off the nail after the appropriate time. This process can be repeated daily over several weeks if required to obtain a complete of the nail infection.

### Example formulation

A formulation of the two component solutions according to the invention is as follows:-

**Table 1: An example of a formulation for the first component containing the active ingredient, hydrogen peroxide, but lacking the photoinitiator.**

| **Ingredient** | **Function** | **Quantity** |
|---|---|---|
| Hydrogen Peroxide Ph Eur * | Active | 1.0 %w/w |
| 2-Acrylamido-2-methyl-1-propanesulfonic acid sodium salt solution (50% solution) (HSE) | Monomer | 30.0%w/w |
| Poly(ethylene glycol) diacrylate (Avergage Mn 700) (HSE) | Cross-linker | 0.2%w/w |
| Hydroxyethyl cellulose PH Eur | Thickener | 1.5%w/w |
| Glycerol Ph Eur | Humectant | 10.0%w/w |

The balance of the formulation is de-ionised water.

**Table 2: An example of a formulation for the second component containing photoinitiator, but lacking the hydrogen peroxide.**

| **Ingredient** | **Function** | **Quantity** |
|---|---|---|
| 2-Hydroxy-2-methylpropiophenone (HSE) | Photoinitator | 1.0 %w/w |
| 2-Acrylamido-2-methyl-1-propanesulfonic acid sodium salt solution (50% solution) (HSE) | Monomer | 30.0%w/w |
| Poly(ethylene glycol) diacrylate (Avergage Mn 700) (HSE) | Cross-linker | 0.2%w/w |
| Hydroxyethyl cellulose PH Eur | Thickener | 1.5%w/w |
| Glycerol Ph Eur | Humectant | 10.0%w/w |

The balance of the formulation is de-ionised water.

In this embodiment of the invention hydrogen peroxide is employed at a nominal concentration of 0.5w/w but, because it is present as a chemotherapeutic agent, in most jurisdictions it is necessary to use only material proved to be of pharmaceutical grade. A suitable commercially available preparation of hydrogen peroxide is known as "PERSYNT^{®}" (Evonik GmbH), in the form of high purity hydrogen peroxide, which is understood to have been optimised for food treatment, fine chemical synthesis as well as for use in the cosmetic and pharmaceutical industries; this material complies with the requirements of the European Pharmacopoeia 7 (except for concentration) and EN DIN 902.

Information supplied by Evonik indicates that PERSYNT is manufactured according to the anthraquinone-autoxidation (AO) process. In this AO process, hydrogen peroxide is produced from hydrogen and atmospheric oxygen, and utilises an anthraquinone derivative, which is circulated, as a "reaction carrier". The crude hydrogen peroxide derived through the AO process is then purified and concentrated. After appropriate stabilization, it is marketed as an aqueous solution at concentrations ranging between 20-35 percent by weight.

Another formulation of the two component solutions according to the invention is as follows:-

**Table 3: An example of a formulation for the first component containing the active ingredient, hydrogen peroxide and thickener.**

| **Ingredient** | **Function** | **Quantity** |
|---|---|---|
| Hydrogen Peroxide Ph Eur * | Active | 1.0 %w/w |
| Hydroxyethyl cellulose PH Eur | Thickener | 1.5%w/w |

The balance of the formulation is de-ionised water.

**Table 4: An example of a formulation for the second component containing photoinitiator, monomer and cross-linker.**

| **Ingredient** | **Function** | **Quantity** |
|---|---|---|
| 2-Hydroxy-2-methylpropiophenone (HSE) | Photoinitator | 1.0 %w/w |
| 2-Acrylamido-2-methyl-1-propanesulfonic acid sodium salt solution (50% solution) (HSE) | Monomer | 60.0%w/w |
| Poly(ethylene glycol) diacrylate (Avergage Mn 700) (HSE) | Cross-linker | 0.4%w/w |
| Hydroxyethyl cellulose PH Eur | Thickener | 1.5%w/w |
| Glycerol Ph Eur | Humectant | 20.0%w/w |

The balance of the formulation is de-ionised water.

### Example 1: In-Vitro Hydrogen Peroxide Release Characteristics

Three formulations were prepared at levels of 0.5, 1.0 and 2.0 wt% active like the formulation in Table 1.

The ability of dual phase formulations to release hydrogen peroxide has been studied *in-vitro.* In an initial study, samples of the 2%w/w active and photoinitiator gels were mixed and then applied to flat sheet "receiver" gel and polymerised *in-situ* using a standardised UV lamp. Samples were then incubated overnight and for 3 days before removing the test gel. The receiver gel was then tested for the presence of hydrogen peroxide using a gel extraction assay, yielding the following results.
- Overnight, more than 70% of the hydrogen peroxide content of the polymerised gel was detected in the receiver gel
- After 3 days' incubation, the receiver gel contained 50-60% of the hydrogen peroxide content of the polymerised gel, suggestive of an equilibrium being established during longer exposure times.

### Example 2: In-Vitro hydrogen peroxide transmission from the gel, through-the-nail

A variation of the example 1 experiment was set up to explore the ability of hydrogen peroxide to penetrate through the nail at concentrations expected to be effective in killing dermatophyte fungal cells. Intact pieces of human nails were glued to the outside of holes (3.2mm diameter) drilled in the centre of petri dishes which were then loaded with starch/iodide agar, taking care to see that the starch iodide gel was in direct contact with the nail piece. The dual phase formulations were mixed in a 1:1 ratio for 20-30 seconds before a single topical application of 0.05-0.06g onto the outer surface of the healthy nail pieces fixed over the hole in each petri dish. The mixed gel was then cured to the nail surface using UV light for 30 seconds. The plates were monitored for colour development, indicative of a reaction between the released hydrogen peroxide and the starch/iodide indicator over a period of 9 hours.

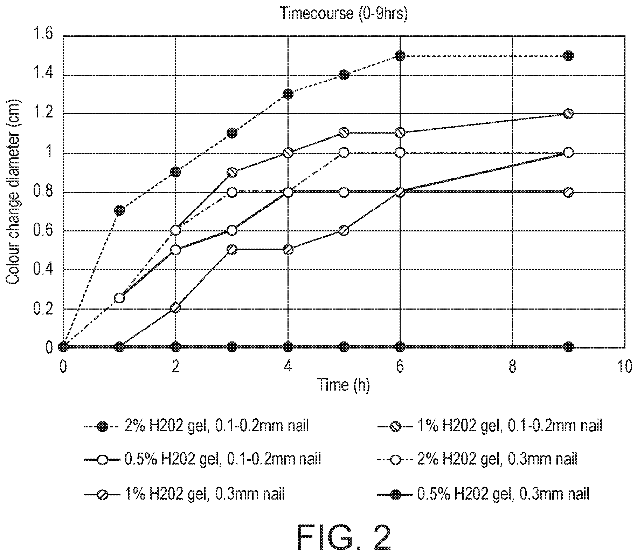

The results, summarised in Figure 2, indicate that colour development, (i.e. release and penetration of hydrogen peroxide through the nail) was fastest and most intense when 2%w/w hydrogen peroxide was applied to healthy nails in the range of 0.1-0.3mm thick. Lower concentrations, 0.5%w/w and 1.0%w/w hydrogen peroxide also showed evidence of significant colour changes, albeit with less intense staining. Consistent with the *in-vitro* efficacy however, no colour changes were observed when the thickest healthy nails (>0.5mm) were used in the assay, irrespective of hydrogen peroxide concentration, indicating that the availability of hydrogen peroxide from single application at the nail bed is likely dependent on the thickness (and therefore permeability) of the healthy nail.

### Example 3: In-Vitro hydrogen peroxide transmission from repeat applications

The aim of this invention is to have sufficient hydrogen peroxide at the nail bed to provide anti-fungal action, whilst avoiding significant collection of oxygen gas underneath the nail, as the pressure of the gas could cause separation of the nail from the nail bed, resulting in pain to the patient. The ideal product therefore provides slow release of hydrogen peroxide, into the nail over several applications, although nail thickness will affect transmission.

In this example repeated applications of the lowest dose of hydrogen peroxide (0.5%w/v gel) were employed in an iodine starch test, using healthy intact nails ranging in thickness from 0.1 - 0.5mm. Either 25µL (equivalent to 0.03mL/cm2) or 125µL (equivalent to 0.15mL/cm2) of the final 0.5%w/v formulation was cured onto the top of the nail pieces and samples were incubated at 25°C overnight. Following incubation, the starch iodide agar was examined for evidence of colour change. Where no colour was observed after the first application, gels were removed from the nail and a fresh gel was reapplied. Samples were then again incubated at 25°C and kept hydrated while monitoring the starch iodide agar for a further 48 hours.

Results after three applications indicate that in the thinnest nails (0.1-0.3mm thick) colour changes are observed after a single application of either 25 or 125µL of 0.5%w/w gel. Additionally, some colour change was observed after a single application in the thickest nail (0.5mm) when 125µL (0.15mL/cm2) was added to the nail. Second applications of the 0.5%w/w gel further enhanced the colour changes observed when 125µL was used and, in addition, colour changes in the 0.4mm thick nails were observed after a second application of only 25µL. Minimal changes were observed after three applications of 25µL of 0.5%w/w gel in the thickest (0.5mm) nails (Table 5).

### Example 4: In-Vitro fungicidal action of hydrogen peroxide transmitted through-the-nail, from gels according to the invention

To confirm that hydrogen peroxide delivered through-the-nail has relevant fungicidal activity, a similar experiment was conducted with fungal killing as the outcome, rather than simply detection of hydrogen peroxide. In this experiment the agar was seeded with *T. rubrum.* A 2%w/w concentration of hydrogen peroxide was used in the final gel applied to the nail pieces, which was polymerised *in-situ* using a standardised UV lamp. In order to compare the efficiency of hydrogen peroxide release from the gel, positive controls were included in the form of simple aqueous solutions of hydrogen peroxide at 0.35%, 3.5% and 35%, which were applied as liquids to the nail surface. Negative controls in the form of water and gels lacking hydrogen peroxide were also included.

The results are summarised in Table 4 below. In overview, these data indicate that the dual phase formulation of this invention, comprising 2%w/w hydrogen peroxide, was capable of penetrating through healthy intact nail pieces and resulted in zones of fungal killing in the underlying agar, although with variable responses. Further investigations indicated that the size of the zones of inhibition was, in large part, related to the thickness of the healthy nail piece used; thicker nail pieces resulted in smaller zones of inhibition, presumably due to slower diffusion and/or impeded penetration through the nail.

By way of confirmation, when the test was repeated without the nail piece, the formulation achieved zones of inhibition comparable with 3.5% hydrogen peroxide solution, indicating that the polymerised gel in itself did not prevent release of the hydrogen peroxide.

**Table 4: Outcome of fungicidal activity of through-the-nail diffusion of hydrogen peroxide from 2%w/w Gel**

| **Treatment** | **Healthy Nail Barrier** | | | **No Nail Barrier** | | |
|---|---|---|---|---|---|---|
| | **Zone of inhibition (mm)** | | | **Zone of inhibition (mm)** | | |
| | Plate 1 | Plate 2 | Plate 3 | Plate 1 | Plate 2 | Plate 3 |
| No Treatment | 0 | - | - | - | - | - |
| Water (Neg control) | 0 | - | - | 0 | - | - |
| 0.35% H₂O₂ Solution | 6 | 0 | 10 | 27 | 25 | 25 |
| 3.5% H₂O₂ Solution | 0 | 30 | 49 | 70 | 72 | 66 |
| 35% H₂O₂ Solution | 35 | No Growth | No Growth | No Growth | No Growth | No Growth |
| Placebo Gel | 0 | 0 | - | 0.5 | 0.5 | - |
| Dual Gel (2%w/w) | 16 | 25 | 2 | 72 | 66 | 80 |

### Example 5: In-Vitro fungicidal action of hydrogen peroxide transmitted through-the-nail, from gels according to the invention in a model more closely resembling the in-vivo situation

The test model of example suffers from a significant drawback, in that the hydrogen peroxide becomes substantially diluted as it diffuses through the agar gel seeded with T. rubrum. Any zone of clearance is limited by this dilution effect. In the in-vivo situation, the fungicidal action is not limited by dilution into a large volume of fungally infected fluid or tissue. In onychomycosis the fungus is largely confined to the nail plate and, possibly, the interface between the nail plate and the nail bed.

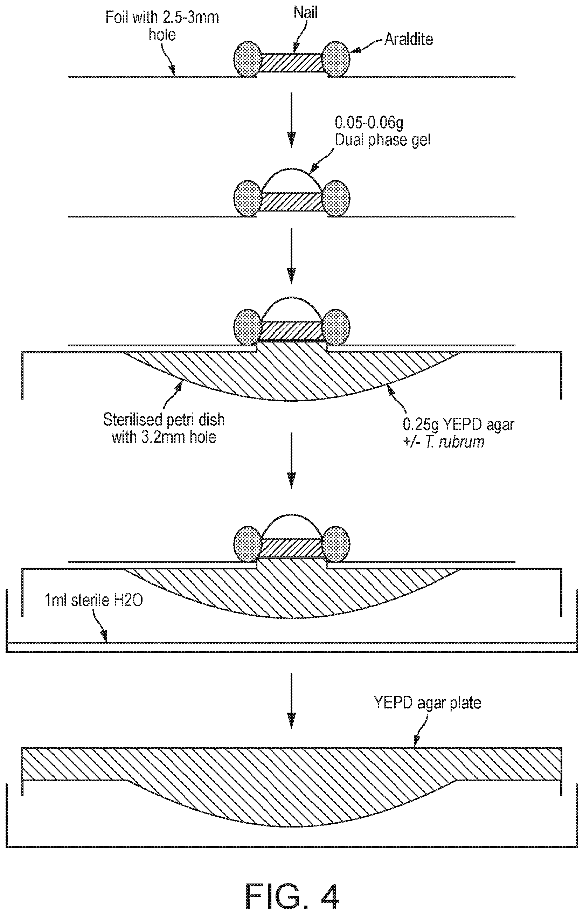

To better simulate the clinical condition of onychomycosis, the test model of Example 4 was modified as shown in Figure 4.

Small quantities (0.05-0.06g) of dual phase gels of varying concentration (0.5%, 1.0% and 2.0% w/w) were cured to the top of a nail clipping using UV light as per the standard model; the primary difference compared to the standard R-SNIPP model was that the 0.25mL of molten YEPD agar (± 104 spores of *T. rubrum*) droplets were allowed to directly contact the underlying side of the nail, rather than relying on diffusion through the agar. The plate was incubated overnight at 25°C; 1mL of sterile water was also added to a petri dish to prevent the agar from drying out. To develop the plates, the agar droplets were transferred to fresh YEPD agar plates and incubated for 4 days at 25°C.

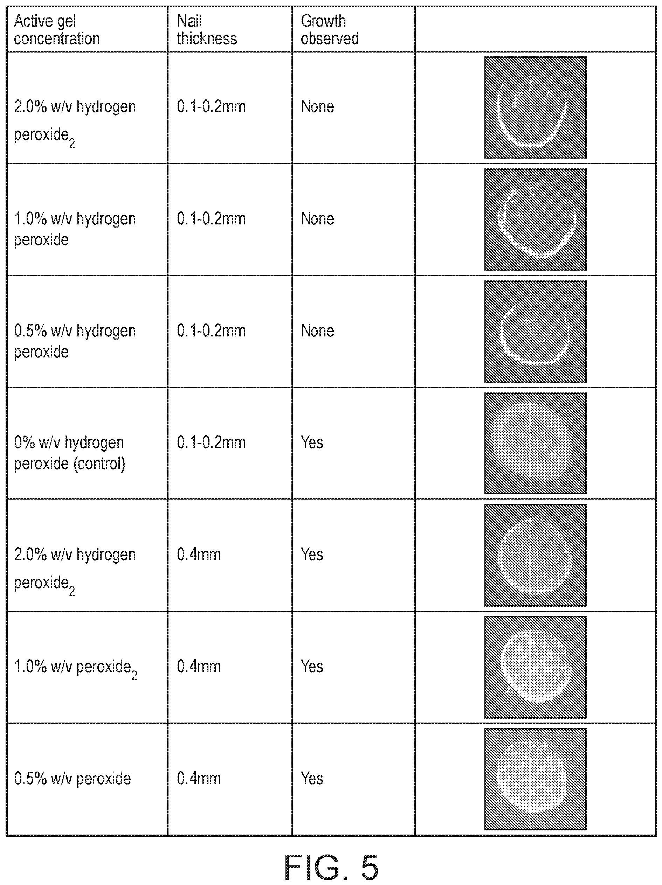

The results from this experiment are shown in Figure 5, in which each result is recorded in words as well as in a photograph of the o.25g target gel after incubation following exposure to through-the-nail hydrogen peroxide.

In this experiment, no growth of *T. rubrum* underlying the nail was observed when dual phase OnyxMyco gels containing hydrogen peroxide from 0.5-2.0%w/v were used on nail clippings of depth 0.1-0.2mm thick. (Table 4). Growth was however recorded with healthy nail clippings in excess of 0.4mm thick, indicating that permeation of hydrogen peroxide through thicker intact healthy nails after a single application may be insufficient to achieve a full fungicidal concentration. Even so, in the 0.4mm thick nail pieces the extent of growth when hydrogen peroxide was included in the formulation was noticeably less than in the zero hydrogen peroxide control.

It is important to recognise that this model assesses only a single application of the invention to a nail piece. In clinical practice, it is intended that applications of this invention are to be repeated daily over the course of several weeks to achieve complete fungal kill. In Example 3, it was clear that repeat applications enhanced the penetration of detectable hydrogen peroxide, even through thicker nails.

A further formulation of the two component solutions according to the invention is as follows:-

**Table 5: An example of a formulation for the first component containing the active ingredient, hydrogen peroxide, but lacking the photoinitiator.**

| **Ingredient** | **Function** | **Quantity** |
|---|---|---|
| Persynt 300 GMP Evonik (Hydrogen Peroxide) | Active | 1.0 %w/w |
| 2-Acrylamido-2-methyl-1-propanesulfonic acid sodium salt solution (50% solution) (AMPS) | Monomer | 60.0%w/w |
| Poly(ethylene glycol) diacrylate (Avergage Mn 700) | Cross-linker | 0.4%w/w |
| Natrosol 250M (Hydroxyethyl cellulose) | Thickener | 1.25%w/w |
| Glycerol | Humectant | 10.0%w/w |
| Disodium EDTA | Peroxide stabiliser | 0.1 |
| pH4 citric acid/sodium citrate buffer, 0.1M | Diluent | 27.25w/w |

If desired, water may be eliminated by increasing the amount of glycerol pro-rata the removed water and
- substituting the acid version of AMPS for the sodium salt of AMPS,
- eliminating EDTA,
- eliminating Natrosol
- replacing Persynt with an equivalent amount of urea-hydrogen peroxide complex Disodium EDTA may be omitted if desired.

Guar gum may be used as a substitute thickener.

**Table 6: An example of a formulation for the second component containing photoinitiator, monomer and cross-linker.**

| **Ingredient** | **Function** | **Quantity** |
|---|---|---|
| 2-Hydroxy-2-methylpropiophenone | Photoinitiator | 1.0 %w/w |
| 8-Hydroxyquinoline | Preservative | 0.4%w/w |
| Natrosol 250M (Hydroxyethyl cellulose) | Thickener | 1.25%w/w |
| Glycerol Ph Eur | Humectant | 10.0%w/w |
| pH4 citric acid/sodium citrate buffer, 0.1M | Diluent | 87.35w/w |

If desired the following preservatives may be used instead:
- Boric acid/sodium borate buffer
- Bronopol 0.02%
- Hexetidine 0.2%
- Potassium sorbate 0.2%

If desired, HMPP may be used at inclusion levels ranging from 0.2% to 0.8% depending on the intensity of UV irradiation available.

## Claims

1. A packaged treatment composition comprising a polymerisable and/or curable composition comprising (1) a packaged first flowable component comprising a source of hydrogen peroxide and (2) a separately packaged second flowable component; the composition comprising a polymerisable monomer or oligomer, a polymer and/or a curing agent, wherein the composition comprises a photoinitiator and wherein the first flowable component is substantially free of photoinitator, the composition capable of forming a solid composition by UV-initiated polymerisation and/or curing following mixing of the first and second flowable components.

2. A packaged treatment composition according to claim 1, wherein the first flowable component is substantially free of polymerisable monomer or oligomer.

3. A packaged treatment composition according to claim 1 or claim 2, wherein the second flowable component is substantially free of polymerisable monomer or oligomer.

4. A packaged treatment composition according to any one of the preceding claims, comprising a curable composition comprising a polymer and a curing agent.

5. A packaged treatment composition according to claim 4, wherein the curing agent is a cross-linking agent.

6. A packaged treatment composition according to claim 4 or claim 5, wherein the first flowable composition is substantially free of curing agent.

7. A packaged treatment composition according to any one of the preceding claims, wherein the first and second flowable components are arranged to produce a hydrated hydrogel upon polymerisation and optionally curing, preferably wherein the polymerisable monomer is an AMPS monomer, forming a poly-AMPS hydrogel upon polymerisation.

8. A packaged treatment composition according to any one of the preceding claims wherein one or both of the flowable components comprises a thickener, preferably a polymeric thickening agent.

9. A packaged treatment composition according to any one of the preceding claims, wherein the first and second compositions are both aqueous solutions.

10. A packaged treatment composition according to any one of the preceding claims, wherein the first and second components are in gel form.

11. A packaged treatment composition according to any one of the preceding claims, wherein the source of hydrogen peroxide comprises pre-formed hydrogen peroxide.

12. A packaged treatment composition according to any one of the preceding claims, wherein the first and second components are packaged to dispense a predefined amount of each of the first and second flowable components.

13. A packaged treatment composition according to claims 11 and 12, wherein the pre-formed hydrogen peroxide is present at a concentration of from 0.2 to 1.5 wt% of the total of the predefined amount of the first and second flowable components.

14. A packaged treatment composition according to any one of the preceding claims, wherein the pH of the first flowable component is less than 5.0.

15. A packaged treatment composition according to claim 12, wherein the packaging provides for mixing of the predefined amount of the first and second flowable components prior to dispensing.

## Patentansprüche

1. Verpackte Behandlungszusammensetzung, umfassend eine polymerisierbare und/oder härtbare Zusammensetzung umfassend (1) eine verpackte erste fließfähige Komponente, die eine Quelle von Wasserstoffperoxid umfasst, und (2) eine getrennt verpackte zweite fließfähige Komponente; wobei die Zusammensetzung ein polymerisierbares Monomer oder Oligomer, ein Polymer und/oder einen Härter umfasst, wobei die Zusammensetzung einen Photoinitiator umfasst und wobei die erste fließfähige Komponente im Wesentlichen frei von Photoinitiator ist, wobei die Zusammensetzung fähig ist, nach Mischen der ersten und der zweiten fließfähigen Komponente durch UV-ausgelöste Polymerisation und/oder Härten eine feste Zusammensetzung zu bilden.

2. Verpackte Behandlungszusammensetzung gemäß Anspruch 1, wobei die erste fließfähige Komponente im Wesentlichen frei von polymerisierbarem Monomer oder Oligomer ist.

3. Verpackte Behandlungszusammensetzung gemäß Anspruch 1 oder Anspruch 2, wobei die zweite fließfähige Komponente im Wesentlichen frei von polymerisierbarem Monomer oder Oligomer ist.

4. Verpackte Behandlungszusammensetzung gemäß einem der vorstehenden Ansprüche, umfassend eine härtbare Zusammensetzung, die ein Polymer und einen Härter umfasst.

5. Verpackte Behandlungszusammensetzung gemäß Anspruch 4, wobei der Härter ein Vernetzer ist.

6. Verpackte Behandlungszusammensetzung gemäß Anspruch 4 oder Anspruch 5, wobei die erste fließfähige Zusammensetzung im Wesentlichen frei von Härter ist.

7. Verpackte Behandlungszusammensetzung gemäß einem der vorstehenden Ansprüche, wobei die erste und die zweite fließfähige Komponente dafür beschaffen sind, durch Polymerisation und gegebenenfalls Härten ein hydratisiertes Hydrogel zu bilden, wobei das polymerisierbare Monomer vorzugsweise ein AMPS-Monomer ist, das durch Polymerisation ein Poly-AMPS-Hydrogel bildet.

8. Verpackte Behandlungszusammensetzung gemäß einem der vorstehenden Ansprüche, wobei eine oder beide der fließfähigen Komponenten ein Verdickungsmittel umfasst, vorzugsweise ein polymeres Verdickungsmittel.

9. Verpackte Behandlungszusammensetzung gemäß einem der vorstehenden Ansprüche, wobei sowohl die erste als auch die zweite Zusammensetzung wässrige Lösungen sind.

10. Verpackte Behandlungszusammensetzung gemäß einem der vorstehenden Ansprüche, wobei die erste und die zweite Komponente in Gelform vorliegen.

11. Verpackte Behandlungszusammensetzung gemäß einem der vorstehenden Ansprüche, wobei die Quelle von Wasserstoffperoxid vorgebildetes Wasserstoffperoxid umfasst.

12. Verpackte Behandlungszusammensetzung gemäß einem der vorstehenden Ansprüche, wobei die erste und die zweite Komponente verpackt sind, um eine vorgegebene Menge von sowohl der ersten als auch der zweiten fließfähigen Komponente auszugeben.

13. Verpackte Behandlungszusammensetzung gemäß Ansprüchen 11 und 12, wobei das vorgebildete Wasserstoffperoxid in einer Konzentration von 0,2 bis 1,5 Gew.-% der Summe der vorgegebenen Mengen der ersten und der zweiten fließfähigen Komponente vorhanden ist.

14. Verpackte Behandlungszusammensetzung gemäß einem der vorstehenden Ansprüche, wobei der pH-Wert der ersten fließfähigen Komponente weniger als 5,0 beträgt.

15. Verpackte Behandlungszusammensetzung gemäß Anspruch 12, wobei die Verpackung Mischen der vorgegebenen Menge der ersten und der zweiten fließfähigen Komponente vor dem Ausgeben ermöglicht.

## Revendications

1. Composition de traitement emballée comprenant une composition polymérisable et/ou durcissable comprenant (1) un premier composant pouvant s'écouler emballé comprenant une source de peroxyde d'hydrogène et (2) un deuxième composant pouvant s'écouler emballé séparément ; la composition comprenant un monomère ou oligomère polymérisable, un polymère et/ou un agent de durcissement, la composition comprenant un photoinitiateur et, le premier composant pouvant s'écouler étant sensiblement exempt de photoinitiateur, la composition étant capable de former une composition solide par polymérisation et/ou durcissement initié(e) par des UV à la suite d'un mélange des premier et deuxième composants pouvant s'écouler.

2. Composition de traitement emballée selon la revendication 1, le premier composant pouvant s'écouler étant sensiblement exempt de monomère ou oligomère polymérisable.

3. Composition de traitement emballée selon la revendication 1 ou la revendication 2, le deuxième composant pouvant s'écouler étant sensiblement exempt de monomère ou oligomère polymérisable.

4. Composition de traitement emballée selon l'une quelconque des revendications précédentes, comprenant une composition durcissable comprenant un polymère et un agent de durcissement.

5. Composition de traitement emballée selon la revendication 4, l'agent de durcissement étant un agent de réticulation.

6. Composition de traitement emballée selon la revendication 4 ou la revendication 5, la première composition pouvant s'écouler étant sensiblement exempte d'agent de durcissement.

7. Composition de traitement emballée selon l'une quelconque des revendications précédentes, les premier et deuxième composants pouvant s'écouler étant agencés pour produire un hydrogel hydraté lors d'une polymérisation et éventuellement d'un durcissement, préférablement le monomère polymérisable étant un monomère d'AMPS, formant un hydrogel de poly-AMPS lors d'une polymérisation.

8. Composition de traitement emballée selon l'une quelconque des revendications précédentes, l'un des composants pouvant s'écouler ou les deux composants pouvant s'écouler comprenant un épaississant, préférablement un agent épaississant polymérique.

9. Composition de traitement emballée selon l'une quelconque des revendications précédentes, les première et deuxième compositions étant toutes deux des solutions aqueuses.

10. Composition de traitement emballée selon l'une quelconque des revendications précédentes, les premier et deuxième composants étant sous forme de gel.

11. Composition de traitement emballée selon l'une quelconque des revendications précédentes, la source de peroxyde d'hydrogène comprenant du peroxyde d'hydrogène pré-formé.

12. Composition de traitement emballée selon l'une quelconque des revendications précédentes, les premier et deuxième composants étant emballés pour diffuser une quantité prédéfinie de chacun des premier et deuxième composants pouvant s'écouler.

13. Composition de traitement emballée selon les revendications 11 et 12, le peroxyde d'hydrogène pré-formé étant présent en une concentration allant de 0,2 à 1,5 % en poids du total de la quantité prédéfinie des premier et deuxième composants pouvant s'écouler.

14. Composition de traitement emballée selon l'une quelconque des revendications précédentes, le pH du premier composant pouvant s'écouler étant inférieur à 5,0.

15. Composition de traitement emballée selon la revendication 12, l'emballage permettant un mélange de la quantité prédéfinie des premier et deuxième composants pouvant s'écouler avant une diffusion.
